Europäisches Patentamt

European Patent Office

Office européen des brevets

(19)

(11) Numéro de publication: **0 219 395**
**A1**

(12) **DEMANDE DE BREVET EUROPEEN**

(21) Numéro de dépôt: **86402045.8**

(22) Date de dépôt: **18.09.86**

(51) Int. Cl.⁴: **A 61 B 5/02**

(30) Priorité: **18.09.85 FR 8513805**

(43) Date de publication de la demande:
**22.04.87 Bulletin 87/17**

(84) Etats contractants désignés:
**AT BE CH DE GB IT LI LU NL SE**

(71) Demandeur: **Becquet, Jacqueline**
**55, Septième Avenue**
**F-60260 Lamorlaye (FR)**

**Denis, Hervé**
**16, rue de Condé**
**F-75006 Paris (FR)**

(72) Inventeur: **Becquet, Jacqueline**
**55, Septième Avenue**
**F-60260 Lamorlaye (FR)**

**Denis, Hervé**
**16, rue de Condé**
**F-75006 Paris (FR)**

(74) Mandataire: **Rodhain, Claude et al**
**Cabinet Claude Rodhain 30, rue la Boétie**
**F-75008 Paris (FR)**

(54) **Appareil de contrôle du rythme cardiaque à détection pléthysmographique.**

(57) Un appareil de surveillance du système cardiovasculaire comporte un détecteur de type pléthysmographique (11), une unité de traitement des signaux obtenus et un dispositif de visualisation (3) délivrant une information écrite et/ou graphique (13).

Le dispositif de mesure pléthysmographique est constitué par au moins un capteur pléthysmographique (11) qui est logé dans la manchette gonflable (12) d'un sphygmomanomètre.

L'appareil peut être utlisé sans aucune contrainte pour l'utilisateur et fournit des indications sur le rythme cardiaque et sur la tension artérielle de l'usager.

FIG.1

EP 0 219 395 A1

**Description**

"Appareil de contrôle du rythme cardiaque à détection pléthysmographique".

L'invention concerne un appareil du type décrit dans le brevet principal et destiné à surveiller le fonctionnement du système cardiovasculaire et qui peut être utilisé sans l'intervention de spécialistes. De tels appareils fournissent des renseignements sur le rythme cardiaque et/ou la tension artérielle; ils sont du type grand public et disposés dans des endroits publics tels que des magasins. Leur fonctionnement simple leur permet d'être utilisés par n'importe quelle personne à qui ils fournissent des renseignements sur le fonctionnement du système cardiovasculaire, renseignements qui peuvent être fournis au médecin traitant et qui peuvent par ailleurs permettre d'alerter l'utilisateur sur l'évolution de son système cardiovasculaire.

L'invention concerne les appareils de ce type qui comportent un détecteur du type pléthysmographique, une unité de traitement des signaux obtenus et un dispositif de visualisation délivrant une information écrite et/ou graphique.

Dans l'appareil décrit dans le brevet principal, la détection pléthysmographique s'effectue sur un doigt de chaque main; pour obtenir un signal utilisable, il est nécessaire d'immobiliser la personne utilisant l'appareil, ce qui crée une contrainte pour l'usager et risque de limiter l'usage de tels appareils.

La présente addition a pour objet un appareil de surveillance du système cardiovasculaire du type précité qui permet d'obtenir une mesure fiable sans créer aucune gêne ni contrainte pour l'usager.

Conformément à la présente addition, le dispositif de mesure pléthysmographique est constitué par au moins un capteur pléthysmographique logé dans une manchette gonflable d'un spygmomanomètre.

Grâce à l'utilisation de la manchette gonflable du sphygmomanomètre on peut maintenir le détecteur pléthysmographique sur le bras de l'usager sans toutefois contraindre ce dernier à rester parfaitement immobile. De plus, l'appareil fournit des indications et sur le rythme cardiaque et sur la tension artérielle de l'usager. Cela permet donc une meilleure surveillance du fonctionnement du système cardiovasculaire.

Selon une autre caractéristique de l'invention, la manchette gonflable du sphygmomanomètre est disposée dans une gouttière qui est elle-même montée à rotation dans toutes les directions sur le châssis de l'appareil. Avantageusement, cette gouttière est fixée sur le châssis par l'intermédiaire d'une articulation à rotule.

De cette manière, l'appareil peut s'adapter à toutes les morphologies des usagers en sorte qu'il n'entraîne pas de gêne pendant la mesure.

Selon encore une autre caractéristique de l'invention, le détecteur pléthysmographique est fixé dans la manchette gonflable de manière à se trouver sous le bras de l'utilisateur. Grâce à ces dispositions, on peut effectuer une mesure sans que l'utilisateur ait à enlever son vêtement; on peut donc effectuer une mesure sur un utilisateur portant une veste par exemple.

Selon encore une autre caractéristique de l'invention, le châssis de l'appareil est monté sur un support constitué de pied verticaux et de montants horizontaux et la forme du châssis est telle qu'il peut être logé dans ledit support en position de transport. Grace à ces dispositions, le support constitue une protection pour le châssis de l'appareil lors du transport.

Le dispositif de visualisation de l'appareil selon l'invention peut comporter un téléviseur associé à un magnétoscope en vue de la diffusion de messages.

D'autres caractéristiques et avantages de l'invention ressortiront de la description qui suit ainsi que des dessins ci-annexés sur lesquels :
- la fig. 1 est une vue en perspective d'un appareil conforme à la présente invention,
- la fig. 2 est une vue de profil de l'appareil de la figure 1.
- la fig. 3 est une vue de détail représentant la gouttière et la manchette gonflable.
- la fig. 4 représente à échelle réduite l'information écrite fournie à l'utilisateur et,
- la fig. 5 représente l'appareil selon l'invention en position de transport.

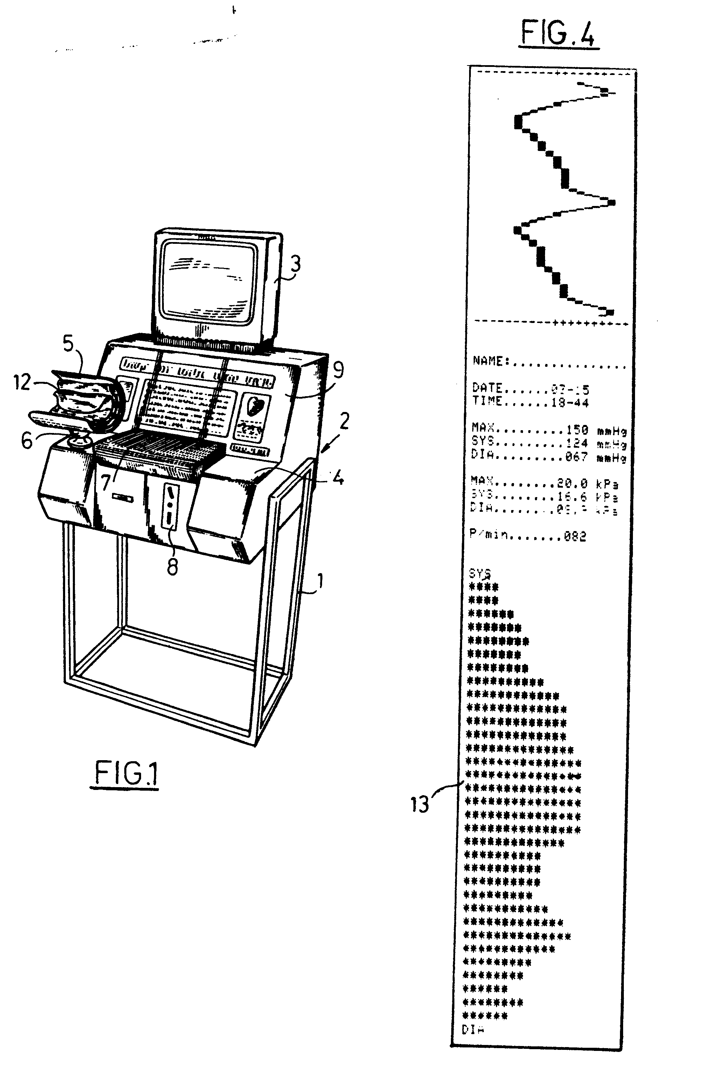

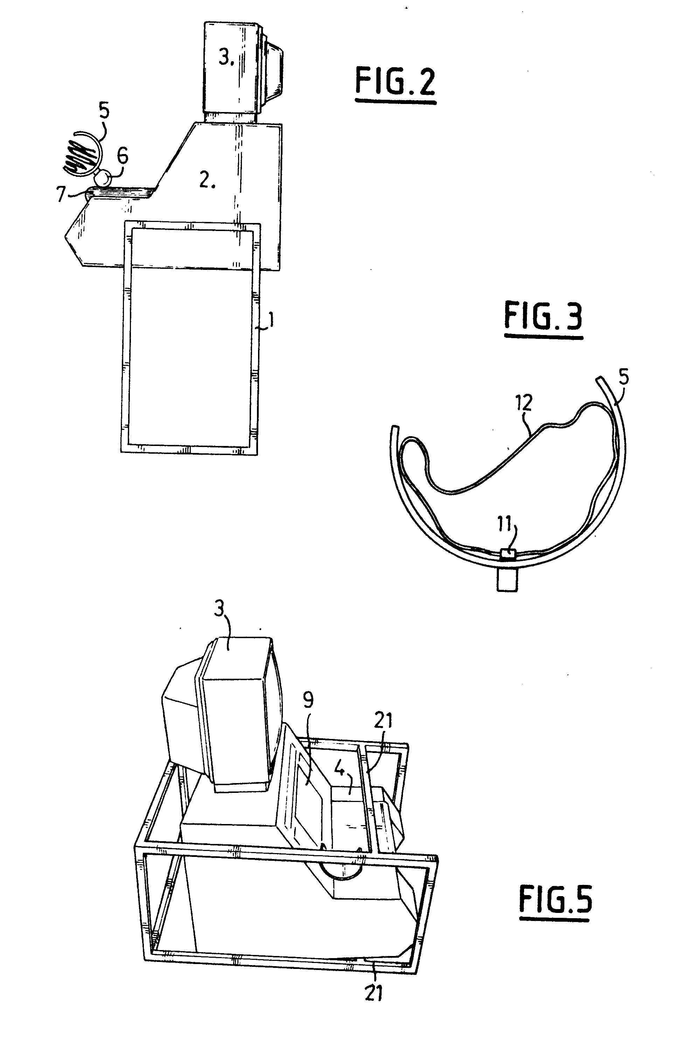

L'appareil décrit à titre d'exemple sur les figures 1 et 2 comporte un support 1 sur lequel est fixé un châssis 2 sur lequel est disposé un téléviseur 3. Le châssis 1 comporte une face horizontale 4 constituant une sorte de tablette sur laquelle est fixée à rotation dans toutes les directions une gouttière 5, par exemple par l'intermédiaire d'une articulation à rotule 6.

Au voisinage de la rotule 6, la tablette 4 est munie d'un plateau 7 servant d'appui pour l'avant bras et surelevé par rapport à la tablet

L'appareil représenté à titre d'exemple est un appareil destiné à l'utilisation dans des lieux publics et il est mis en oeuvre par l'intermédiaire d'un monnayeur 8.

Le châssis 2 comporte une face frontale 9 verticale ou inclinée sur laquelle sont portées des indications, en particulier les instructions pour l'utilisation de l'appareil.

Conformément à l'invention, comme représenté en particulier à la figure 3, l'appareil comporte un seul détecteur pléthysmographique 11 qui est logé dans une manchette gonflable 12 d'un tensiomètre ou manomètre. Ce dernier comporte de manière usuelle un dispositif de gonflage et de dégonflage ainsi qu'un manomètre. De ce fait, l'appareil selon l'invention permet à la fois de déterminer le rythme cardiaque et la tension artérielle de l'utilisateur.

Le fonctionnement de l'appareil qui vient d'être décrit est le suivant.

L'utilisateur passe tout d'abord son bras dans la manchette gonflable 12 qui est alors à l'état dégonflé. Comme indiqué plus haut, il n'est pas nécessaire de dénuder le bras, la mesure pouvant s'effectuer sur un bras revêtu d'une manche de veste.

L'utilisateur prend une position confortable en appuyant son avant bras sur le plateau 7; avantagement, un siège est disposé devant l'appareil afin que l'utilisateur soit parfaitement à son aise. Comme indiqué plus haut, le fonctionnement de l'appareil est déclenché après introduction de pièces dans le monnayeur 8 par une pression sur un bouton de commande.

Tout d'abord, la manchette 12 est gonflée jusqu'à une valeur de pression telle que le vaisseau soit comprimé et que le sang ne circule plus quelle que soit la pression artérielle de l'utilisateur. Ensuite la manchette est déglonflée de manière régulière, par exemple à une cadence de 0,3 kPa/s ; aussitôt que la pression du sang provenant du coeur devient supérieure à la pression exercée par la manchette, le sang circule et cela est détecté par le détecteur pléthysmographique 11 puisque le vaisseau se dilate et que cela se traduit par une modification de la pression qui est mesurée par le détecteur 11. L'amplitude de l'onde de pression atteint son maximum lorsque la pression régnant dans la manchette est égale à la pression artérielle diastolique. Lorsque la pression de la manchette descend en dessous de la pression diastolique, la circulation sanguine progressivement redevient normale.

La pression dans la manchette est mesurée de façon continue avec un détecteur tel qu'un manomètre; par ailleurs l'onde de pression produite par la circulation du sang du côté refoulement de la manchette est mesuré par le détecteur pléthysmographique 11; le signal obtenu est filtré dans un circuit différentiel et il est utilisé en tant que critère pour la mémorisation des valeurs systolique et diastolique de la pression artérielle. Pour la systole, le critère est la première apparition d'une onde de pression suivie de deux autres qui présentent une augmentation d'amplitude d'au moins 10% par rapport à la précédente. La diastole est mémorisée, lorsque l'amplitude de la variation de pression est descendue à 50% de sa valeur maximale.

Pour compenser des impulsions parasites éventuelles lors de la mesure, par exemple si la manchette se déplace sur l'utilisateur, on ne prend pas en compte des modifications de deux amplitudes de pression successives pour lesquelles la variation est supérieure à 100%; dans ce cas au lieu de prendre en compte la deuxième amplitude présentant cette variation supérieure à 100%, on prend en compte la valeur précédente avec une pondération qui est fonction de la pente de progression.

Ce procédé présente l'avantage de présenter une grande sécurité contre les parasites et une meilleure reproductibilité de la mesure de la pression artérielle. Ce procédé est en particulier supérieur sur ces points aux méthodes de Krotkow et de Riva Rocci. Ce procédé présente également l'avantage que dans des environnements bruyants on peut réaliser une mesure sans problème. Les avantages sont en particulier dûs à l'utilisation du détecteur pléthysmographique.

Les ondes de pulsations sont imprimées sous forme de traits de longueur variable de manière à réaliser une courbe enveloppe comme représenté en 13 sur la Figure 5 qui représente l'information délivrée par une imprimante à chaque utilisateur.

Sur cette figure on voit tout d'abord l'enregistrement graphique d'une onde d'impulsion qui correspond par exemple à la huitième impulsion mesurée; cette courbe graphique comporte des parties positives et des parties négatives; les parties positives correspondent à la circulation du sang à partir du coeur et les parties négatives à la circulation en retour du sang. La fiche délivrée par l'imprimante faisant partie du dispositif d'affichage indique le nom, la date, l'heure, la pression maximale de la manchette, la pression systolique et la pression diastolique, ces pressions étant exprimées d'une part en millimètres de mercure et en kilopascale (aPa). La fiche délivrée fournit ensuite la fréquence de battement du coeur puis la courbe enveloppe précitée.

Selon une autre caractéristique de l'invention, comme représentée sur la figure 4, la forme du châssis 2 est telle qu'il peut être logé dans le support 1 dans sa position de transport. Dans cette position, le châssis 2 repose sur une des traverses horizontales 21 sur lesquelles il est fixé lorsqu'il est en position d'utilisation. La profondeur du châssis 1 est donc inférieure à la hauteur du support 1 et la hauteur du châssis est inférieure à la profondeur dudit support.

Sur les figures, on voit un téléviseur 3 qui est commandé par un magnétoscope qui est inclu dans le châssis 2; cet ensemble du téléviseur et du magnétoscope sert à envoyer des messages tels que des messages publicitaires.

La description ci-dessus d'un exemple de réalisation de l'invention n'a été fournie qu'à titre illustratif et nullement limitatif et il est évident que l'on peut y apporter des modifications ou variantes sans pour autant sortir du cadre de la présente invention.

## Revendications

1°. Appareil de contrôle du rythme cardiaque à détection pléthysmographique conforme aux revendications 12 et 14 du brevet principal, comportant un dispositif de mesure pléthysmographique, une unité de traitement des signaux obtenus constitué par un microprocesseur comportant une mémoire de microprogrammation et un dispositif de visualisation délivrant une information écrite et graphique comprenant un écran de télévision, caractérisé en ce que le dispositif de mesure pléthysmographique est constitué par au moins un capteur pléthysmographique (11) qui est logé dans une manchette gonflable (12) d'un sphygmomanomètre.

2°. Appareil de surveillance selon la revendication 1, caractérisé en ce que ladite manchette gonflable (12) est disposée dans une gouttière (5) montée en rotation dans toutes les directions sur le châssis (2) de l'appareil.

3°. Appareil de surveillance selon la revendication 2, caractérisé en ce que ladite gouttière (5) est fixée sur le châssis (2) au moyen d'une articulation à rotule (6).

4°. Appareil de surveillance selon la revendication 1, caractérisé en ce que le capteur pléthysmographique (11) est fixé dans la manchette gonflable (12) de manière à se trouver sous le bras de l'utilisateur.

5°. Appareil de surveillance selon la revendication 1, caractérisé en ce que le châssis (2) de l'appareil est monté sur un support (1) constitué de pieds verticaux et de montants horizontaux (21) et en ce que la forme du châssis (2) est telle qu'il peut être logé dans ledit support (1) en position de transport.

6°. Appareil de surveillance selon la revendication 3, caractérisé en ce que le châssis (5) de l'appareil comporte en partie centrale et en surélévation, un plateau horizontal (7) disposé au voisinage de l'articulation à rotule (6).

7°. Appareil de surveillance selon la revendication 1, caractérisé en ce que le dispositif de visualisation comporte un téléviseur (3) associé à un magnétoscope en vue de la diffusion de messages.

FIG.4

```
NAME:...............

DATE......07-15
TIME......18-44

MAX.........150  mmHg
SYS.........124  mmHg
DIA.........067  mmHg

MAX........20.0  kPa
SYS........16.6  kPa
DIA........0... kPa

P/min.......082

SYS
****
****
*****
******
*******
*******
********
*********
**********
***********
************
*************
*************
**************
***************
****************
****************
*****************
*****************
****************
***************
**************
*************
************
***********
**********
*********
********
*******
******
*****
****
DIA
```

FIG.1

**FIG.2**

**FIG.3**

**FIG.5**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl.4) |
|---|---|---|---|
| Y | EP-A-0 098 222 (J. BECQUET et al.) <br> * Abrégé; page 4, ligne 1 - page 5, ligne 4; page 5, lignes 23-27; page 8, lignes 16-30; page 10, revendication 1; page 11, revendications 6,12,14; figures 1-3 * | 1 | A 61 B 5/02 |
| A | | 5-7 | |
| | --- | | |
| Y | FR-A-2 262 952 (J.-C. DARRAS) <br> * Page 1, lignes 1-4,25-49; page 2, lignes 78-90; figures 1-4 * | 1 | |
| | --- | | |
| A | US-A-4 105 021 (W.J. WILLIAMS et al.) <br> * Abrégé; colonne 3, lignes 10-20,31-44; colonne 5, ligne 42 - colonne 6, ligne 2; figures 1,2,5,6 * | 1,2,4, 5 | DOMAINES TECHNIQUES RECHERCHES (Int. Cl.4) <br><br> A 61 B |
| | --- | | |
| A | US-A-4 312 359 (T.A. OLSON) <br> * Abrégé; colonne 4, ligne 65 - colonne 5, ligne 26; colonne 6, lignes 12-18; figures 1-3 * | 1,2,6 | |
| | --- | | |
| A | DE-A-1 466 899 (SIEMENS AG) <br> * Page 2, ligne 15 - page 3, ligne 8; page 3, lignes 16-23; figures 1,2 * | 1,3,4 | |
| | ---      -/- | | |

Le présent rapport de recherche a été établi pour toutes les revendications

| Lieu de la recherche | Date d'achèvement de la recherche | Examinateur |
|---|---|---|
| LA HAYE | 11-12-1986 | RIEB K.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

## DOCUMENTS CONSIDERES COMME PERTINENTS

| Catégorie | Citation du document avec indication, en cas de besoin. des parties pertinentes | Revendication concernée | CLASSEMENT DE LA DEMANDE (Int Cl.4) |
|---|---|---|---|
| A | DE-A-2 513 445 (J.P. GOUS)<br>* Page 6, lignes 16-18; figure 2 * | 3 | |
| | ----- | | |
| | | | **DOMAINES TECHNIQUES RECHERCHES (Int. Cl.4)** |

Le présent rapport de recherche a été établi pour toutes les revendications

| Lieu de la recherche | Date d'achèvement de la recherche | Examinateur |
|---|---|---|
| LA HAYE | 11-12-1986 | RIEB K.D. |